# FASCICULE DE BREVET EUROPEEN

(11) **EP 1 557 194 B1**
(45) Date de publication et mention de la délivrance du brevet: **15.10.2008**
(21) Numéro de dépôt: 05290130.3
(22) Date de dépôt: 21.01.2005
(51) Int. Cl.: A61N 1/05

(54) **Sonde de defibrillation monocorps**
Einstückige Defibrillationssonde
Single-piece defibrillation sonde

(30) Priorité: 22.01.2004 FR 0400577
(43) Date de publication de la demande: 27.07.2005
(73) Titulaire: ELA MEDICAL, 92541 Montrouge (FR)
(72) Inventeur: Ollivier, Jean-François, 91190 Villiers le Bacle (FR); Bessoule, Frédéric, 91360 Villemoisson sur Orge (FR)
(74) Mandataire: Dupuis-Latour, Dominique

(56) Documents cités:
- US-A1- 2001 018 607

## Description

L'invention concerne les "dispositifs médicaux implantables actifs" tels que définis par la directive 90/385/CEE du 20 juin 1990 du Conseil des communautés européennes.

Elle concerne plus particulièrement la famille des appareils chargés de délivrer au coeur des impulsions électriques de haute énergie (c'est-à-dire dépassant notablement l'énergie fournie pour la simple stimulation) pour tenter de mettre fin à une tachyarythmie. Ces dispositifs sont appelés "défibrillateurs implantables" ou " cardioverteurs implantables", étant entendu que l'invention couvre aussi bien les défibrillateurs/cardioverteurs/stimulateurs ou les défibrillateurs/stimulateurs implantables.

Ces dispositifs sont constitués de deux ensembles, à savoir un générateur d'impulsions et une sonde ou un système de sondes.

Le générateur d'impulsions est chargé de surveiller l'activité cardiaque et de générer des impulsions de haute énergie quand le coeur présente une arythmie ventriculaire susceptible d'être traitée. Quand cette énergie est comprise entre 0,1 et 10 J environ, on désigne cette thérapie sous le nom de "cardioversion" et le choc électrique est appelé "choc de cardioversion". Quand cette énergie est supérieure à 10 J environ, le choc électrique est alors appelé "choc de défibrillation".

À ce générateur sont connectées une ou plusieurs sondes munie(s) d'électrodes dont le rôle est de distribuer au coeur cette énergie de façon appropriée.

L'invention concerne le cas particulier où le générateur est relié à une sonde dite "monocorps", qui est une sonde unique portant les différentes électrodes permettant de délivrer le choc de défibrillation ou de cardioversion.

Ces électrodes de choc se présentent sous la forme de bobinages de fil métallique supportés par l'extrémité tubulaire distale de la sonde et destinés à venir en contact avec les tissus à l'endroit où doit être appliquée l'énergie de cardioversion ou de défibrillation. Le bobinage est relié à un fil conducteur parcourant la sonde sur sa longueur jusqu'à la borne correspondante du connecteur situé à l'extrémité proximale.

Ces sondes monocorps comportent le plus souvent deux électrodes de choc, à savoir une électrode dite "supraventriculaire", qui sera positionnée dans la veine cave supérieure pour appliquer le choc à l'oreillette, et une deuxième électrode, ventriculaire, située plus près de l'extrémité distale de la sonde.

Ces sondes sont généralement des sondes de type "isodiamètre", c'est-à-dire qu'elles présentent le même diamètre sur toute la longueur de la partie distale destinée à être implantée dans le réseau veineux, précisément pour faciliter cette implantation - ainsi qu'une explantation ultérieure. Ceci signifie que la surface extérieure des bobinages formant les électrodes de choc affleure la surface extérieure, isolante du reste de la gaine, de manière à ne présenter aucun saut de diamètre sur la longueur implantée.

La réalisation de ces sondes est relativement délicate, compte tenu de la présence des bobinages, des contraintes de continuité du diamètre et de la nécessité de réaliser la liaison électrique à l'intérieur du corps de la sonde avec le fil conducteur permettant la délivrance de l'énergie de choc.

La technique employée jusqu'à présent pour fabriquer ces sondes consiste à réaliser une pluralité de tronçons tubulaires de gaine emboîtables les uns à la suite des autres et à mettre en place les bobinages et les raccorder électriquement à leur conducteur interne au fur et à mesure de l'emboîtement des différents tronçons du tube de sonde.

Cette structure, qui permet de répondre aux contraintes spécifiques de fabrication de ces sondes, a cependant pour inconvénient de créer des zones de faiblesse mécanique et/ou électrique aux endroits des raccordements des différents tronçons, notamment des courts-circuits sur le conducteur haute tension d'amenée de l'énergie de choc. Or, en pratique, il a été constaté que les ruptures du tube support isolant se produisent toujours aux endroits des raccords entre les différents tronçons de gaine, du fait des zones de faiblesse localement créées à l'endroit de ces raccords.

De plus, cette structure en tronçons emboîtés implique un processus de fabrication relativement complexe et long, notamment du fait de la nécessité de coller les tronçons successifs.

Les US-A-6 374 142 et WO-A-02/087689 décrivent de telles sondes monocorps isodiamètre réalisées à partir de tronçons de gaine successifs emboîtés.

Les caractéristiques du préambule de la revendication 1 sont connues de l'enseignement du document WO-A-02/087689. US2001/0018607 dévoile une sonde dont certaines structures souples sans solution de continuité axiale au lieu des bobines. Toutefois ces dites structures ne sont pas tubulaires.

L'un des buts de l'invention est de remédier à ces inconvénients, en proposant une autre structure pour la partie distale d'une sonde de défibrillation monocorps, qui ne présente pas de zone de faiblesse au voisinage des bobinages et qui par ailleurs, du point de vue de la technologie, puisse être fabriquée de façon simple et rapide.

La sonde de l'invention est une sonde monocorps de défibrillation du type connu décrit ci-dessus, c'est-à-dire avec un corps de sonde comprenant à son extrémité distale un coeur de gaine tubulaire en matériau souple isolant, supportant à sa périphérie au moins un bobinage de fil métallique formant électrode de choc pour l'application d'une énergie de défibrillation ou de cardioversion, ce bobinage étant électriquement relié à un fil conducteur de liaison s'étendant longitudinalement dans une lumière interne du coeur de gaine.

De façon caractéristique de l'invention, le coeur de gaine s'étend axialement sans solution de continuité dans la (les) région(s) supportant le(s) bobinage(s).

Très avantageusement, le coeur de gaine comporte localement un évidement traversant situé au voisinage d'au moins l'une des extrémités du bobinage, et il est prévu en outre un insert en matériau conducteur, de dimensions homologues dudit évidement et logé dans celui-ci, cet insert étant électriquement relié, côté intérieur, au fil conducteur et, côté extérieur, à l'extrémité correspondante du bobinage.

En particulier, le coeur de gaine peut comporter un évidement au voisinage de chacune des extrémités du bobinage, et il comporte alors également une fente longitudinale traversante reliant les deux évidements et s'étendant radialement depuis la surface externe du coeur de gaine jusqu'à la lumière interne, de manière à permettre, par déformation élastique du matériau du coeur de gaine de part et d'autre de la fente, l'introduction dans les évidements et dans la lumière interne de l'ensemble formé par l'extrémité terminale du fil conducteur préalablement pourvue des deux inserts auxquels elle a été mécaniquement et électriquement reliée.

Dans un mode de mise en oeuvre préférentiel, il est en outre prévu une bague de jonction pour la liaison mécanique et électrique de l'insert au bobinage, cette bague étant une bague cylindrique en matériau conducteur, avec une surface interne apte à coopérer avec une partie tournée vers l'extérieur de l'insert, et une surface externe comportant une partie de liaison apte à coopérer avec une partie tournée vers l'intérieur de l'extrémité du bobinage.

Cette bague peut notamment comporter, dans la région de la surface interne apte à coopérer avec l'insert, une partie d'assemblage apte à permettre une solidarisation mécanique et électrique de la bague à l'insert. La partie d'assemblage est de préférence une partie comportant un orifice traversant apte à permettre une solidarisation de la bague à l'insert par soudage depuis l'extérieur. De plus, le diamètre de la partie d'assemblage est supérieur au diamètre de la partie de liaison, la différence des diamètres étant sensiblement égale au double de l'épaisseur du bobinage, de manière que la surface extérieure apparente de la bague soit sensiblement en affleurement de la surface extérieure du bobinage.

Il est avantageusement prévu en outre sur la sonde une enveloppe exteme en matériau souple isolant gainant le coeur de gaine sur la longueur de celui-ci à l'exception de la région du bobinage, le diamètre de l'enveloppe externe étant sensiblement égal au diamètre du bobinage. Dans ce cas, la bague peut comporter également, à l'opposé de la partie de liaison, une partie d'emmanchement recevant l'extrémité de l'enveloppe exteme adjacente au bobinage.

Pour l'assemblage, l'insert peut comporter, côté intérieur, un manchon, orienté axialement, de sertissage de l'insert au fil conducteur.

De préférence, l'espace compris entre les parois radiales de la fente, ainsi que le volume interne de la lumière dans la région de la fente, sont garnis d'un matériau d'étanchéité électriquement isolant, notamment une résine polymère durcissable telle qu'une colle silicone.

On va maintenant décrire un exemple de réalisation de l'invention, en référence aux dessins annexés.

La figure 1 est une vue d'ensemble, en perspective, d'une sonde monocorps de défibrillation selon l'invention.

La figure 2 est une vue perspective, agrandie, de l'extrémité proximale de la gaine tubulaire, à l'endroit où cette dernière se termine pour s'élargir et se diviser en une pluralité de conducteurs reliés à un organe de connexion.

La figure 3 est une vue perspective, éclatée, montrant le coeur de gaine et les éléments qui y seront insérés pour permettre ultérieurement la connexion au bobinage de défibrillation.

La figure 4 est une vue en coupe détaillée de la partie de sonde à l'endroit du bobinage de défibrillation, montrant les différents éléments internes et la manière dont est effectuée la liaison électrique à ce bobinage.

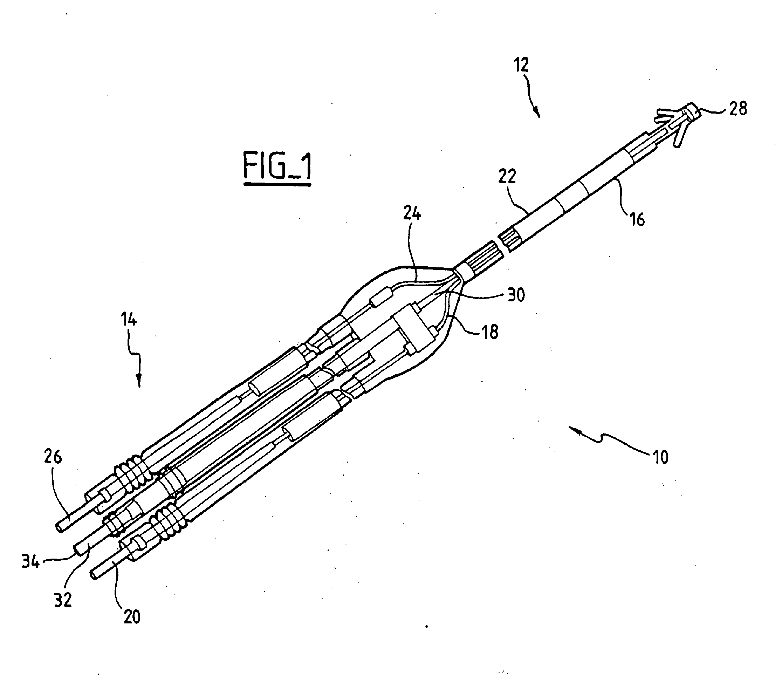

Sur la figure 1, la référence 10 désigne de façon générale une sonde monocorps dont la partie distale 12 est destinée à être introduite par le réseau veineux dans les deux cavités auriculaire et ventriculaire, de manière à y détecter l'activité cardiaque et appliquer en tant que de besoin un choc de défibrillation ou de cardioversion. Cette sonde est munie à son extrémité proximale 14 de divers éléments de connexion à un générateur approprié, par exemple un générateur du type *Defender* ou *Lyra* fabriqué par ELA Medical, Montrouge, France.

La sonde 10 porte une première électrode de choc 16, destinée à venir se placer dans le ventricule droit et constituant par exemple la borne négative d'application du potentiel de défibrillation ou de cardioversion. Cette électrode de choc ventriculaire est reliée par un conducteur de liaison 18 à une borne 20 de connexion au générateur, avantageusement une borne au standard DF-1.

La sonde 10 porte également sur sa partie distale 12 une deuxième électrode de choc 22, qui est une électrode dite "supraventriculaire" destinée à être positionnée dans la veine cave supérieure pour l'application d'un choc à l'oreillette ; cette électrode supraventriculaire 22 est reliée par un autre conducteur 24 à une autre borne 26 de connexion au générateur, de préférence également au standard DF-1.

La sonde 10 est également pourvue d'une électrode d'extrémité 28, qui est une électrode de détection/stimulation destinée à être positionnée au fond de la cavité ventriculaire droite. Cette électrode 28 est reliée par un conducteur 30 à une borne 32 de liaison au stimulateur, avantageusement au standard IS-1.

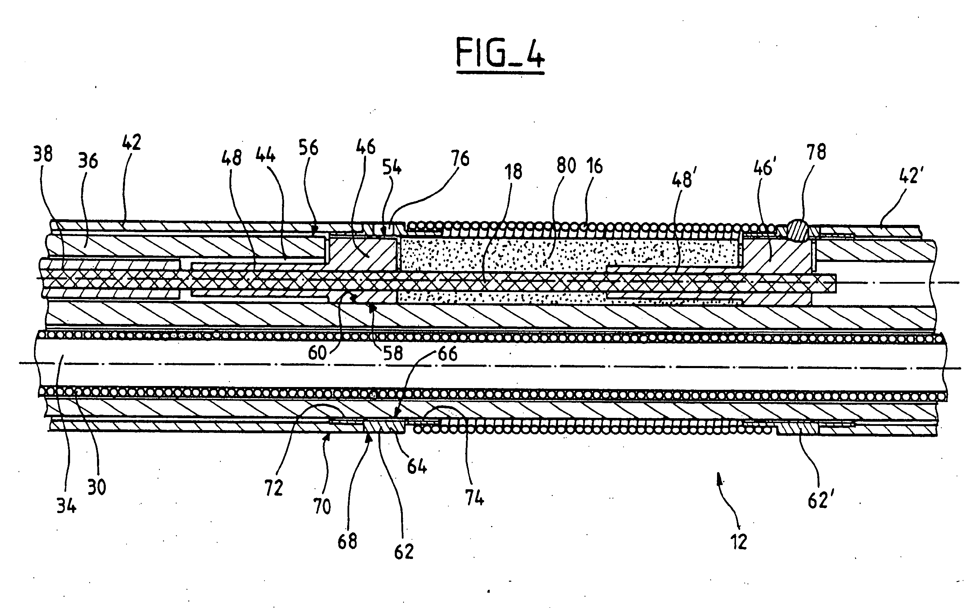

Comme on peut le voir sur la figure 4, le conducteur 30 est, de manière en elle-même classique, un conducteur creux, par exemple un conducteur bobiné interne, avec en son centre une lumière 34 permettant l'introduction d'un stylet pour le guidage de l'extrémité 12 par le praticien dans le réseau veineux au moment de l'implantation de la sonde.

Le potentiel de défibrillation peut être appliqué entre l'électrode supraventriculaire 22 et le corps du générateur, ou bien entre l'électrode ventriculaire 16 et le corps du générateur, ou bien entre ces deux électrodes 16 et 22, en mode bipolaire.

La configuration que l'on vient de décrire (deux électrodes de défibrillation et une électrode de stimulation) n'est cependant pas limitative, et l'invention est aussi bien applicable au cas d'une sonde pourvue d'une seule électrode de défibrillation bobinée, ou ne comprenant pas d'électrode distale de stimulation, ou comprenant deux électrodes de stimulation (pour une stimulation en mode bipolaire, notamment), ou plus.

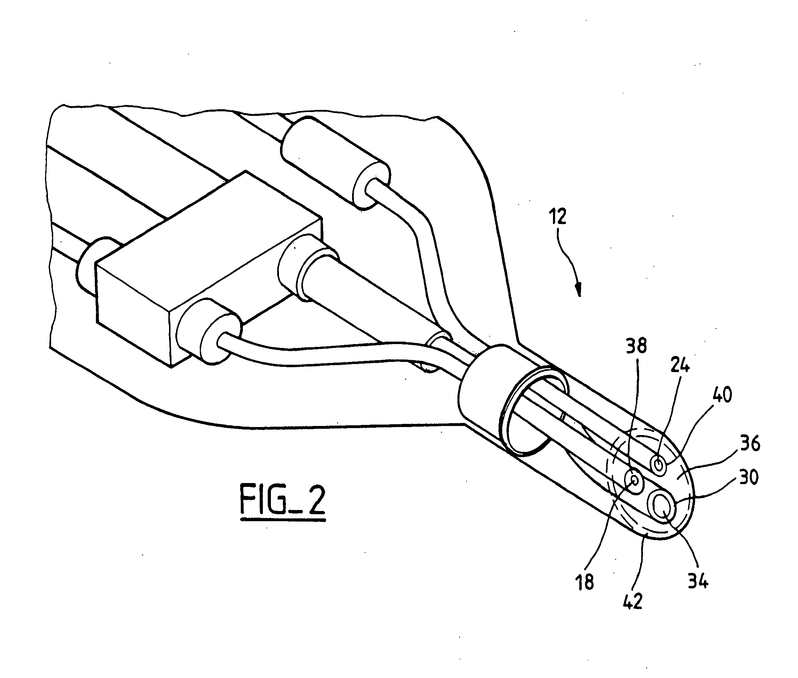

Les figures 2 et 4 montrent plus précisément la configuration des trois conducteurs 18, 24 et 30 dans l'extrémité tubulaire distale 12 de la sonde.

Ces conducteurs sont logés dans des lumières respectives d'un coeur de gaine tubulaire monobloc 36 en matériau souple isolant tel qu'un silicone. Les conducteurs 18 et 24, qui doivent transmettre l'énergie de défibrillation ou de cardioversion, sont des microcâbles possédant leur isolant propre, respectivement 38 et 40, par exemple en ETFE.

Le matériau silicone constituant le coeur de gaine 36 présente d'excellentes propriétés de résistance à la fatigue ; en revanche, il serait difficile à faire pénétrer tel quel dans le réseau veineux. Pour cette raison, le coeur de gaine 36 est enveloppé extérieurement par une gaine 42 en matériau à faible coefficient de frottement, par exemple en polyuréthanne.

L'invention concerne plus particulière la manière dont est constituée la sonde au voisinage des bobinages 16 et 22 constituant les électrodes de choc.

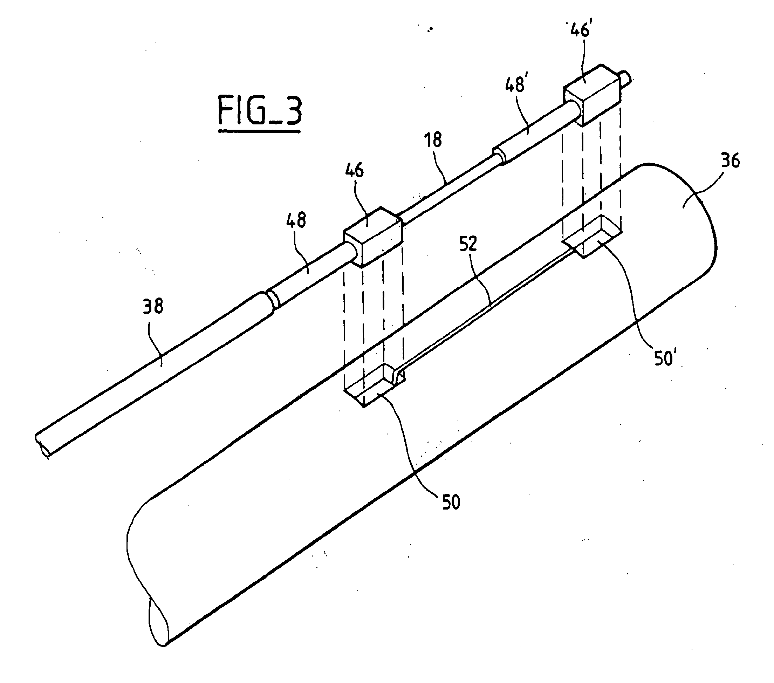

Les figures 3 et 4 illustrent cette structure particulière pour le bobinage 16, la structure étant la même pour l'autre bobinage 22, que l'on ne décrira pas en détail.

De façon caractéristique de l'invention, le coeur de gaine 36 est un tube monobloc, sans solution de continuité sur toute la longueur de la partie distale 12, notamment dans la région des bobinages 16 et 22, et ce grâce à une structure particulière du système de liaison électrique du bobinage à son conducteur correspondant situé à l'intérieur de ce coeur de gaine.

Ainsi, comme illustré figures 3 et 4, le conducteur 18 destiné à alimenter le bobinage 16 est pourvu de deux pièces métalliques 46, 46' qui feront fonction d'inserts, ces inserts 46 étant solidarisés mécaniquement, et électriquement reliés, au fil conducteur 18 par sertissage de manchons 48, 48' sur une longueur dénudée émergeant de l'isolant 38.

Il est en effet souhaitable d'avoir une liaison électrique du conducteur 18 avec les deux extrémités du bobinage 16, afin de produire un champ électrique le plus homogène possible entre ces deux extrémités au moment de l'application de l'énergie de défibrillation ou de cardioversion. Si le bobinage est alimenté par ses deux extrémités, la densité de courant sera en effet mieux répartie, évitant ainsi les risques de brûlure des tissus environnants : pour un choc de défibrillation pouvant nécessiter l'application d'une énergie allant jusqu'à 40 joules, la tension de crête peut atteindre 750 V, et pour cette tension l'homogénéité du champ électrique au moment du choc est une contrainte importante à prendre en compte à la conception de la sonde.

Comme illustré figure 3, le coeur de gaine 36 comporte localement deux évidements 50, 50', qui sont des évidements qui traversent le coeur de gaine 36 depuis la surface extérieure de ce dernier jusqu'à la lumière 44 (figure 4) recevant le conducteur 18.

Ces deux évidements 50, 50' sont par ailleurs réunis par une fente longitudinale 52 (figure 3) s'étendant le long d'une génératrice du coeur de gaine tubulaire 36 et, radialement, depuis la surface externe de ce dernier jusqu'à la lumière interne 44 recevant le conducteur 18.

Les dimensions intérieures de ces évidements 50, 50' sont homologues des dimensions extérieures des inserts 46, 46', afin que ceux-ci puissent être entièrement logés dans les évidements, leur surface supérieure 54 (figure 4) venant affleurer la surface supérieure 56 du coeur de gaine 36. Côté intérieur, la face inférieure 58 de l'insert 46 repose sur la surface de fond 60 de la lumière interne 44.

La liaison électrique et mécanique des insert 46, 46', et donc du conducteur 18, avec le bobinage 16, est réalisée par l'intermédiaire de bagues de jonction 62, 62'.

Cette bague de jonction présente une partie centrale 64 dont la surface intérieure 66 vient en contact avec la surface supérieure 54 de l'insert 46.

La surface extérieure 68 de la partie centrale 64, quant à elle, présente un diamètre approximativement égal au diamètre externe du bobinage 16 et au diamètre externe de la gaine polyuréthanne 42 ; de cette manière, la surface externe 70 de cette dernière vient en affleurement de la surface externe 68 de la bague, assurant ainsi la configuration isodiamètre recherchée.

Du côté opposé au bobinage, la bague 62 comporte une partie de moindre diamètre 72 destinée à venir s'emmancher à force dans l'extrémité intérieure de la gaine extérieure 42. Côté bobinage, la bague de liaison 62 comporte une partie de moindre diamètre 74 destinée à venir s'emmancher à force dans l'extrémité intérieure du bobinage 16.

Enfin, pour assurer la solidarisation électrique et mécanique de l'insert 46 à la bague de liaison 62 (et donc au bobinage 16), la partie centrale 64 de la bague est pourvue d'un orifice traversant 76 permettant de réaliser depuis l'extérieur un point de soudage 78 (comme illustré à droite sur la figure 4), de préférence un point de soudage laser.

Enfin, sous le bobinage 16, le vide subsistant autour du conducteur 18 et des divers éléments attenants est garni d'un matériau d'étanchéité électriquement isolant, par exemple une résine polymère durcissable telle qu'une résine silicone.

On va maintenant décrire la manière de réaliser une telle structure de sonde avec continuité mécanique du coeur de gaine dans la région supportant l'électrode.

Tout d'abord, le coeur de gaine 36 est préparé avec sa gaine extérieure 42 uniquement dans la région proximale, c'est-à-dire sur la partie droite de la figure 4 ; cette gaine externe s'arrête donc au voisinage de l'évidement 50 côté proximal (à gauche sur les figures 3 et 4).

Séparément (par exemple sur une autre chaîne de préparation) l'isolant 38 du conducteur 18 est dénudé côté distal sur une longueur appropriée, pour y sertir les deux plots 46, 46' à la distance voulue, au moyen des manchons 48, 48'. L'ensemble obtenu est celui illustré en partie supérieure de la figure 3.

Le conducteur 18 est alors enfilé par son extrémité proximale (c'est-à-dire celle opposée aux plots) dans la lumière 44 sur toute la longueur de celle-ci, via l'ouverture 50 du coeur de gaine 36, en laissant dépasser côté distal l'extrémité libre avec les inserts 46, 46'. L'ensemble formé de cette longueur de fil avec les inserts 46, 46' est alors complètement introduit à l'intérieur du coeur de gaine 36, en logeant les deux inserts 46, 46' dans les deux évidements homologues 50, 50', la partie de conducteur reliant ces deux inserts étant introduite par déformation élastique du matériau du coeur de gaine de part et d'autre de la fente 52. Une fois l'ensemble ainsi introduit, les manchons 48, 48' et le conducteur 18 trouvent leur place à l'intérieur de la lumière 44 et les deux lèvres de la fente 52 peuvent donc reprendre leur forme initiale. L'ensemble est maintenu définitivement en place de manière étanche par injection locale, via la fente 52, d'une masse de résine silicone durcissable (référencée 80 sur la figure 4) qui vient donc remplir la lumière 44 à l'endroit de la fente 52 et des évidements 50, 50', avec obturation étanche de la lumière 44 de part et d'autre de l'ensemble ainsi constitué.

Les étapes suivantes consistent à, successivement :
- enfiler la bague 62,
- l'emmancher dans la partie de gaine extérieure 42 située côté proximal (à gauche sur la figure 4),
- enfiler le bobinage 16,
- emmancher l'extrémité proximale du bobinage sur la bague 62,
- enfiler la bague 62',
- l'emmancher sur l'extrémité distale du bobinage 16,
- enfiler la partie de gaine 42' côté distal,
- l'emmancher sur la bague 62'.

L'ensemble est ainsi mécaniquement assemblé. L'opération est répétée identiquement pour l'autre bobinage. Des points de soudage laser permettent de parfaire la liaison électrique et mécanique des bagues 62, 62' d'une part aux extrémités du bobinage 16 (dans la zone d'emmanchement 74), et d'autre part aux inserts respectifs 46, 46'.

## Revendications

1. Une sonde de défibrillation monocorps (10), avec un corps de sonde comprenant à son extrémité distale (12) un coeur de gaine tubulaire (36) en matériau souple isolant, supportant à sa périphérie au moins un bobinage (16 ; 22) de fil métallique formant électrode de choc pour l'application d'une énergie de défibrillation ou de cardioversion, ce bobinage étant électriquement relié à un fil conducteur de liaison (18) s'étendant longitudinalement dans une lumière interne (44) du coeur de gaine,
sonde **caractérisée en ce que** le coeur de gaine (36) s'étend axialement sans solution de continuité dans la (les) région(s) supportant le(s) bobinage(s) (16 ; 22).

2. La sonde monocorps de la revendication 1, où le coeur de gaine (36) comporte localement un évidement traversant (50 ; 50') situé au voisinage d'au moins l'une des extrémités du bobinage, et où il est prévu en outre un insert (46 ; 46') en matériau conducteur, de dimensions homologues dudit évidement (50 ; 50') et logé dans celui-ci, cet insert étant électriquement relié, côté intérieur, au fil conducteur (18) et, côté extérieur, à l'extrémité correspondante du bobinage.

3. La sonde monocorps de la revendication 1, où le coeur de gaine (36) comporte un évidement (50, 50') au voisinage de chacune des extrémités du bobinage, et comporte également une fente longitudinale traversante (52) reliant les deux évidements et s'étendant radialement depuis la surface externe (56) du coeur de gaine jusqu'à la lumière interne (44), de manière à permettre, par déformation élastique du matériau du coeur de gaine de part et d'autre de la fente, l'introduction dans les évidements et dans la lumière interne (44) de l'ensemble formé par l'extrémité terminale du fil conducteur (18) préalablement pourvue des deux inserts (46, 46') auxquels elle a été mécaniquement et électriquement reliée.

4. La sonde monocorps de la revendication 2, où il est en outre prévu une bague de jonction (62) pour la liaison mécanique et électrique de l'insert au bobinage, cette bague étant une bague cylindrique en matériau conducteur, avec une surface interne (66) apte à coopérer avec une partie tournée vers l'extérieur (54) de l'insert, et une surface externe comportant une partie de liaison (74) apte à coopérer avec une partie tournée vers l'intérieur de l'extrémité du bobinage.

5. La sonde monocorps de la revendication 4, où la bague comporte, dans la région de la surface interne apte à coopérer avec l'insert, une partie d'assemblage (62) apte à permettre une solidarisation mécanique et électrique de la bague à l'insert.

6. La sonde monocorps de la revendication 5, où la partie d'assemblage est une partie comportant un orifice traversant (76) apte à permettre une solidarisation de la bague (62) à l'insert (46) par soudage depuis l'extérieur.

7. La sonde monocorps de la revendication 5, où le diamètre de la partie d'assemblage (62) est supérieur au diamètre de la partie de liaison (74), la différence des diamètres étant sensiblement égale au double de l'épaisseur du bobinage, de manière que la surface extérieure apparente (68) de la bague soit sensiblement en affleurement de la surface extérieure du bobinage.

8. La sonde monocorps de la revendication 7, où il est en outre prévu une enveloppe externe (42) en matériau souple isolant gainant le coeur de gaine (36) sur la longueur de celui-ci à l'exception de la région du bobinage, le diamètre de l'enveloppe externe étant sensiblement égal au diamètre du bobinage.

9. La sonde monocorps de la revendication 8, où la bague comporte également, à l'opposé de la partie de liaison (74), une partie d'emmanchement (72) recevant l'extrémité de l'enveloppe externe (42) adjacente au bobinage.

10. La sonde monocorps de la revendication 2, où l'insert (46) comporte, côté intérieur, un manchon (48), orienté axialement, de sertissage de l'insert (46) au fil conducteur (18).

11. La sonde monocorps de la revendication 3, où l'espace compris entre les parois radiales de la fente, ainsi que le volume interne de la lumière (44) dans la région de la fente, sont garnis d'un matériau d'étanchéité (80) électriquement isolant.

12. La sonde monocorps de la revendication 11, où le matériau d'étanchéité (80) est une résine polymère durcissable telle qu'une colle silicone.

## Claims

1. A single-body defibrillation probe (10), with a probe body including at its distal end (12) a tubular sheath core (36) made out of an insulating flexible material, supporting at its periphery at least one winding (16 ; 22) of metallic wire forming a shock electrode for the application of a defibrillation or cardioversion energy, said winding being electrically connected to a conducting wire for connection (18) which extends longitudinally in an internal lumen (44) of the sheath core,
said probe being **characterised in that** the sheath core (36) axially extends without interruption in the area(s) supporting the winding(s) (16 ; 22).

2. The single-body probe of claim 1, wherein the sheath core (36) locally includes a through cavity (50 ; 50') located in the vicinity of at least one of the ends of the winding, and wherein there is further provided an insert (46 ; 46') made out of a conductive material, with dimensions homologous to said through cavity (50 ; 50') and housed in the latter, said insert being electrically connected, on the inner side, to the conductive wire (18) and, on the outer side, to the corresponding end of the winding.

3. The single-body probe of claim 1, wherein the sheath core (36) comprises a cavity (50, 50') in the vicinity of each of the ends of the winding, and further comprises a longitudinal through slit (52) connecting the two cavities and radially extending from the outer surface (56) of the sheath core to the internal lumen (44), whereby allowing, by elastic deformation of the material of the sheath core on both sides of the slit, the introduction into the cavities and in the internal lumen (44) of the assembly formed by the terminal end of the conductive wire (18) provided beforehand with the two inserts (46, 46') to which it had been mechanically and electrically connected.

4. The single-body probe of claim 2, wherein it is further provided a junction ring (62) for the mechanical and electrical connection of the insert to the winding, this ring being a cylindrical ring in a conductive material, with an inner surface (66) adapted to cooperate with an outside facing region (54) of the insert, and an outer surface comprising a connecting region (74) adapted to cooperate with an inside facing region of the end of the winding.

5. The single-body probe of claim 4, wherein the ring includes, in the area of the inner surface adapted to cooperate with the insert, an assembling region adapted to enable a mechanical and electric binding of the ring to the insert.

6. The single-body probe of claim 5, wherein said assembling region is a region comprising a through hole (76) adapted to enable a binding of the ring (62) to the insert (46) by a welding made from the outside.

7. The single-body probe of claim 5, wherein the diameter of the assembling region (62) is greater than the diameter of the connecting region (74), the difference of the diameters being about twice the thickness of the winding, whereby the outer visible surface (68) of the ring is approximately flush with the outer surface of the winding.

8. The single-body probe of claim 7, wherein it is further provided an outer envelope (42) made out of a flexible insulated material sheathing said sheath core (36) over the length thereof but in the area of the winding, the diameter the outer envelope being approximately equal to the diameter of the winding.

9. The single-body probe of claim 8, wherein the ring further includes, at the opposite side of the connecting region (74), a shafting region (72) receiving the end of the outer envelope (42) adjacent to the winding.

10. The single-body probe of claim 2, wherein the insert (46) comprises, on the inner side, an axially oriented sleeve (48) for crimping the insert (46) to the conductive wire (18).

11. The single-body probe of claim 3, wherein the interval between radial walls of the slit, as well as the internal volume of the lumen (44) in the area of the slit, are provided with an electrically insulating sealing material (80).

12. The single-body probe of claim 11, wherein said sealing material (80) is a polymeric hardening resin such as a silicone glue.

## Patentansprüche

1. Einkörper-Defibrilierungssonde (10) mit einem Sondenkörper, welcher an seinem entfernten Ende (12) ein Kernstoff von rohrförmigem Mantel (36) aus isolierendem weichen Material umfasst, welcher in seinem Umfang zumindest eine Wicklung (16, 22) aus Metalldraht aufnimmt, welcher eine Schockelektrode für die Anwendung einer Defibrilations- oder Kardioversionsenergie bildet, wobei diese Wicklung mit einem leitenden Verbindungsdraht (18) elektrisch verbunden ist, welcher sich längs in einer internen Öffnung (44) des Mantelkernstoffs ausdehnt, wobei die Sonde **dadurch** charakterisiert ist, dass sich der Mantelkernstoff (36) axial ohne Kontinuitätslösung in der (den) Region(en) ausdehnt, welche die Wicklung(en) (16, 22) aufnimmt.

2. Einkörpersonde nach Anspruch 1, in welcher der Kernstoff des Mantels (36) zumindest lokal eine Aussparung (50, 50') in Längsrichtung einschließt, welche sich in Nachbarschaft zu zumindest einer der Enden der Spule befindet und wo außerdem ein Einsatz (46, 46') aus leitendem Material vorgesehen ist, welcher homologe Dimension entsprechend der Aussparung (50, 50') hat, und in dieser angeordnet ist, wobei dieser Einsatz auf seiner Innenseite elektrisch mit dem leitenden Draht (18) verbunden ist, und auf der Außenseite mit dem entsprechenden Ende de Spule verbunden ist.

3. Einkörpersonde nach Anspruch 1, in welcher der Kernstoff (36) eine Aussparung (50, 50') in Nachbarschaft jedes der Enden der Spule umfasst und auch einen longitudinalen durchquerenden Schlitz (52) einschließt, welcher die Aussparung verbindet, und sich radial von der äußeren Oberfläche (56) des Kernstoffs bis zu der internen Öffnung (44) in der Weise ausdehnt, um durch elastische Deformierung des Materials des Mantelherzens auf den beiden Seiten des Schlitzes, die Einführung der Gesamtheit, welche durch das abschließende Ende des leitenden Drahts (18) gebildet wird, zuvor versehen mit zwei Einsätzen (46, 46'), mit welchen sie mechanisch und elektrisch verbunden wurde, in die Aussparung und in die interne Öffnung (44) zu ermöglichen.

4. Einkörpersonde nach Anspruch 2, wo außerdem ein Verbindungsring (62) für die mechanische und elektrische Verbindung des Einsatzes zur Windung vorgesehen ist, wobei dieser Ring ein zylindrischer Ring aus leitendem Material mit einer inneren Oberfläche (66) ist, welche dazu geeignet ist, mit einem nach außen gewandten Abschnitt (54) des Einsatzes zusammenzuwirken, und mit einer äußeren Oberfläche, welche einen Verbindungsabschnitt (44) umfasst, welcher geeignet ist, um mit einem Abschnitt des Endes der Wicklung, welcher nach innen gewandt ist, zusammenzuwirken.

5. Einkörpersonde nach Anspruch 4, in welcher der Ring in der Region der internen Oberfläche, welche geeignet ist, mit dem Einsatz zusammenzuwirken, einen Montageabschnitt (62) umfasst, welcher dazu geeignet ist, eine mechanische und elektrische Befestigung des Rings an dem Einsatz zu ermöglichen.

6. Einkörpersonde nach Anspruch 5, in welcher der Montageabschnitt ein Abschnitt ist, welcher eine durchgehende Öffnung (76) umfasst, welche dazu geeignet ist, eine Befestigung des Rings (62) an dem Einsatz (46) durch Schweißen von außen zu ermöglichen.

7. Einkörpersonde nach Anspruch 5, in welcher der Durchmesser des Montageabschnitts (62) größer als der Durchmesser des Verbindungsabschnitts (74) ist, wobei der Unterschied der Durchmesser deutlich gleich oder doppelt der Dicke der Spule ist, in der Weise, dass die außen erscheinende Oberfläche (68) des Rings im Ausbiss der äußeren Oberfläche der Wicklung ist.

8. Einkörpersonde nach Anspruch 7, in welcher unter anderem eine äußerer Umschlag (42) aus weichem isolierenden Material vorgesehen ist, welcher den Kernstoff des Mantels (36) auf der Länge dieses mit Ausnahme der Region der Wicklung ummantelt, wobei der Durchmesser des äußeren Umschlags deutlich gleich dem Durchmesser der Wicklung ist.

9. Einkörpersonde nach Anspruch 8, in welcher der Ring auch auf der Gegenseite des Verbindungsabschnitts (74) auch einen Einpassungsabschnitt (72) aufweist, welcher das Ende der äußeren Ummantelung (42), welche angrenzend an die Wicklung ist, aufnimmt.

10. Einkörpersonde nach Anspruch 2, in welcher der Einsatz (46) auf der Innenseite einen Stutzen (48) umfasst, welcher axial von der Bördelung des Einsatzes (46) zum leitenden Draht (18) ausgerichtet ist.

11. Einkörpersonde nach Anspruch 3, in welcher der Raum, welcher zwischen den radialen Wänden und dem Schlitz enthalten ist, sowie das innere Volumen der Öffnung (44) in der Region des Schlitzes mit einem elektrisch isolierenden Dichtungsmaterial (80) versehen sind.

12. Einkörpersonde nach Anspruch 11, in welcher das Dichtungsmaterial (80) ein haltbares Harzpolymer so wie ein Silikonkleber ist.
